(19) Europäisches Patentamt
European Patent Office
Office européen des brevets

(11) Veröffentlichungsnummer : **0 135 076**
**B1**

(12) # EUROPÄISCHE PATENTSCHRIFT

(45) Veröffentlichungstag der Patentschrift : **29.07.87**

(51) Int. Cl.[4] : **C 07 D237/18**, A 01 N 43/58

(21) Anmeldenummer : **84108980.8**

(22) Anmeldetag : **28.07.84**

(54) **Pyridazinon-Derivate, Verfahren zu ihrer Herstellung un ihre Verwendung als Fungizide.**

(30) Priorität : **10.08.83 DE 3328770**

(43) Veröffentlichungstag der Anmeldung : **27.03.85 Patentblatt 85/13**

(45) Bekanntmachung des Hinweises auf die Patenterteilung : **29.07.87 Patentblatt 87/31**

(84) Benannte Vertragsstaaten : **AT BE CH DE FR GB IT LI NL SE**

(56) Entgegenhaltungen : **CHEMICAL ABSTRACTS, Band 68, Nr. 1, 1. Januar 1968, Seite 275, Nr. 2906c, Columbus, Ohio, US**

(73) Patentinhaber : **BASF Aktiengesellschaft Carl-Bosch-Strasse 38 D-6700 Ludwigshafen (DE)**

(72) Erfinder : **Graf, Hermann, Dr. Ginsterstrasse 15 D-6704 Mutterstadt (DE)**
Erfinder : **Rentzea, Costin, Dr. Richard-Kuhn-Strasse 1-3 D-6900 Heidelberg (DE)**
Erfinder : **Richarz, Winfried, Dr. Koenigsberger Strasse 5 D-6084 Stockstadt (DE)**
Erfinder : **Froehlich, Helmut, Dr. Dirmsteiner Weg 35 D-6700 Ludwigshafen (DE)**
Erfinder : **Ammermann, Eberhard, Dr. Sachsenstrasse 3 D-6700 Ludwigshafen (DE)**
Erfinder : **Pommer, Ernst-Heinrich, Dr. Berliner Platz 7 D-6703 Limburgerhof (DE)**

**EP 0 135 076 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Pyridazinon-Derivate, Verfahren zu ihrer Herstellung, Fungizide, die diese Verbindungen als Wirkstoffe enthalten, sowie Verfahren zur Bekämpfung von Pilzen.

Es ist bekannt, N-Trichlormethylthio-tetrahydrophthalimid als Fungizid in der Landwirtschaft sowie im Obst- und Gartenbau einzusetzen (vgl. z. B. Wegler, « Chemie der Pflanzenschutz- und Schädlingsbekämpfungsmittel », Bd. 2, S. 109 ff, Springer Verlag Berlin/Heidelberg/New York, 1970). Das bekannte Mittel ist jedoch nur vor der Infektion verwendbar und seine Wirkung genügt bei niedrigen Aufwandmengen den Anforderungen der Praxis nicht.

Es wurde nun gefunden, daß Pyridazinon der Formel I

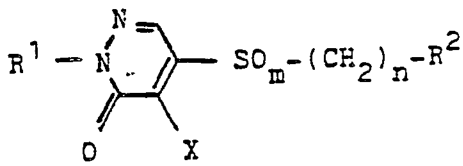

(I)

worin

$R_1$ Wasserstoff ; $C_1$-$C_8$-Alkyl, ggf. ein- oder mehrfach durch Halogen substituiert ; $C_3$-$C_7$-Cycloalkyl ; Phenyl, ggf. ein- oder mehrfach, gleich oder verschieden substituiert durch Halogen, $C_1$-$C_4$-Alkyl, Mono-, Di- oder Trihalogen-$C_1$-$C_3$-Alkyl, Mono-, Di- oder Trihalogen-$C_1$-$C_3$-Alkoxy, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkoxycarbonyl, Mono- oder Di-($C_1$-$C_4$-Alkyl)-carbamoyl, Cyan, Thiocyan oder Nitro ; Carboxyl ; Hydroxymethyl ; $—CH_2O—COR^3$, wobei $R^3$ $C_1$-$C_6$-Alkyl bedeutet ; $—CH_2O[CH_2CH_2O]_pR^4$, wobei $R^4$ $C_1$-$C_4$-Alkyl oder Phenyl und p 1, 2 oder 3 bedeutet ;

$R^2$ Phenyl, ggf. ein- oder mehrfach, gleich oder verschieden substituiert durch Halogen, $C_1$-$C_4$-Alkyl (ggf. ein- oder mehrfach mit Halogen substituiert), $C_1$-$C_4$-Alkoxy (ggf. ein- oder mehrfach mit Halogen substituiert), $C_1$-$C_4$-Alkylthio, $C_1$-$C_4$-Alkylsulfinyl, $C_1$-$C_4$-Alkylsulfonyl, Mono- oder Di-($C_1$-$C_4$-Alkyl)-carbamoyl, Cyan, Thiocyan oder Nitro ; Vinyl oder 2-Propenyl ;

X Halogen

m 1 oder 2 und

n 1, 2, 3 oder 4

bedeuten, eine gute fungizide Wirksamkeit besitzen und leicht herstellbar sind.

In der Formel I bedeutet $R^1$ vorzugsweise einen Methyl- oder Phenylrest, $R^2$ einen Phenylrest, X Chlor oder Brom, m die Zahl zwei und n die Zahlen eins oder zwei.

Die neuen Pyridazinon-Derivate der Formel I lassen sich herstellen, indem man 4,5-Dihalogen-pyridazin-2(2H)one der Formel II

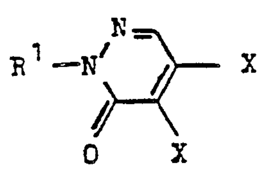

(II)

mit Mercaptanen der Formel III

$$R^2—(CH_2)_n—SH \qquad (III),$$

wobei $R^1$, $R^2$, X und n die oben angegebene Bedeutung haben, in Anwesenheit einer Base, beispielsweise in einem organischen Solvens umsetzt und die so erhaltenen Thioverbindungen IV

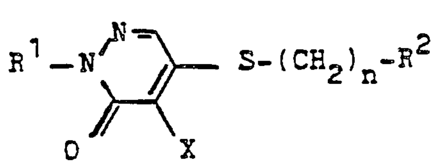

(IV)

ggf. in einem anorganischen oder organischen Solvens, zu Verbindungen der Formel I oxidiert.

Verbindungen des Typs IV sind bereits bekannt, jedoch werden bei den bekannten Herstellungsmethoden zwei Stufen benötigt, ausgehend von der Dihalogenverbindung II oder sie liefern unerwünschte Nebenprodukte. In Chem. Abstr. 68, 2906 c (1968) wird die Herstellung von 4-Chlor-5-mercapto-2-phenyl-pyridazin-3(2H)-on (V), ausgehend von 4,5-Dichlor-2-phenyl-pyridazin-3(2H)-on (II, $R^1 = C_6H_5$, X = Cl) und Natriumhydrogensulfid, beschrieben, das mit Benzylchlorid und Natrium- sowie Ammoniumhydroxid zum gewünschten 5-Benzylthio-4-chlor-2-phenyl-pyridazin-3(2H)-on umgesetzt wird.

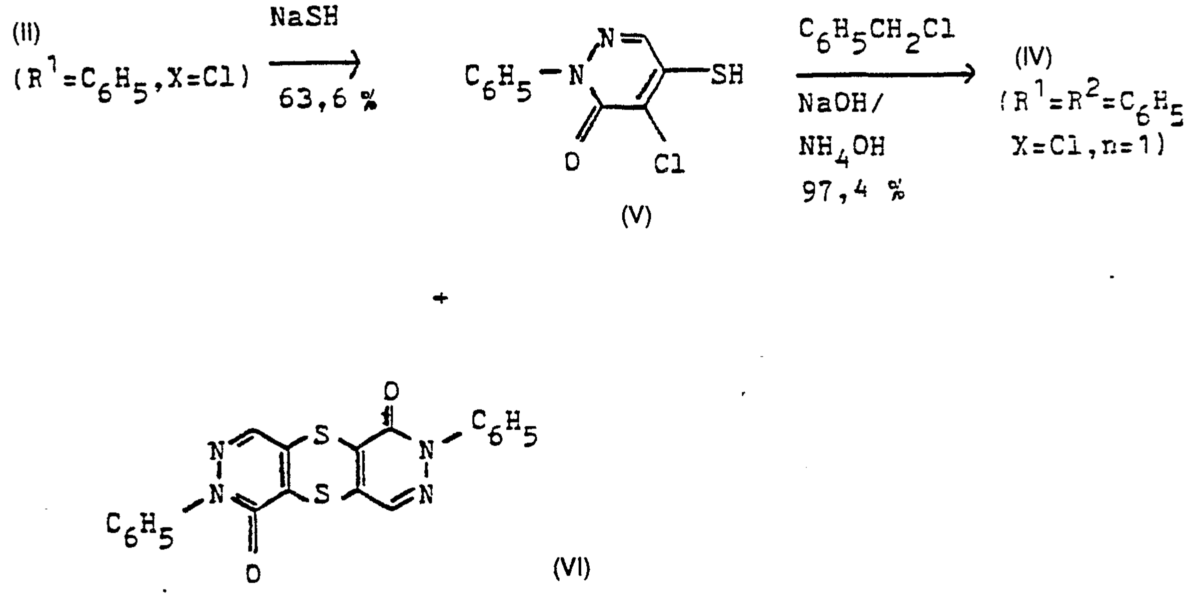


Bei der ersten Stufe entsteht jedoch 2,7-Diphenyl-dipyridazo-[4,5-b : 4,5-e]-1,4-dithiin-1,6-dion (VI) als unerwünschtes Nebenprodukt (vgl. R.N. Castle, K. Kaji und D. Wise, J. Heterocyclic Chem. 3, 541 bis 543 (1966) sowie K. Kaji, M. Kuzuya und R.N. Castle, Chem. Pharm. Bull. 18, 147 bis 156 (1970).

Eine weitere bekannte Methode (vgl. R.F. Meyer, J. Heterocyclic Chem. 6, 407 bis 408 (1969)) liefert, ausgehend von 4,5-Dichlor-2-methyl-pyridazin-3(2H)-on (II, $R^1 = CH_3$, X = Cl), in einer Stufe das gewünschte 5-Benzylthio-4-chlor-2-methyl-pyridazin-3(2H)-on (IV, $R^1 = CH_3$, $R^2 = C_6H_5$, X = Cl, n = 1, jedoch nur in 37 % Ausbeute ; zudem ist das benötigte S-Benzyl-isothiouronium-chlorid nicht kommerziell erhältlich, sondern muß speziell hergestellt werden.

Wie eigene Versuche ergaben, besteht zudem die Möglichkeit der Entstehung der unerwünschten Produkte VII und VIII.

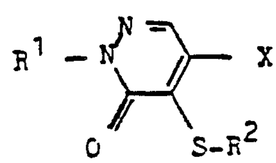

(VII)

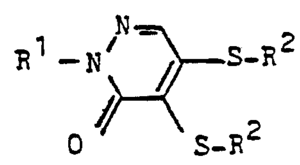

(VIII)

Es wurde nun gefunden, daß 5-thiosubstituierte Pyridazinone des Typs IV in einer Stufe ohne störende Nebenreaktionen aus 4,5-dihalogensubstituierten Pyridazinonen II erhalten werden können.

Hierzu werden 4,5-dihalogensubstituierte Pyridazinone des Typs II mit Mercaptanen III in Anwesenheit einer Base und in einem nichtnucleophilen Lösungsmittel bei Temperaturen zwischen — 40 °C und + 100 °C, vorzugsweise aber zwischen — 20 °C und + 50 °C, zur Reaktion gebracht.

Als Basen werden Amine bevorzugt wie Trimethylamin, Triethylamin, Triisobutylamin, Dimethylethylamin, Ethyldiisopropylamin, N,N-Dimethylanilin, N,N-Diethylanilin oder 2-(Diethylamino)-ethanol.

Als Lösungsmittel seien Alkohole wie Isopropanol, sec-Butanol, tert-Butanol, sec-Isoamylalkohol und tert-Amylalkohol genannt.

Zur Oxidation der 5-thiosubstituierten Pyridazinone IV zu 5-sulfinyl-substituierten (I, m = 1) bzw. 5-sulfonylsubstituierten (I, m = 2) Pyridazinonen können beispielsweise verwendet werden :

Peroxide, wie Wasserstoffperoxid, tert.-Butylhydroperoxid ; Persäuren, wie Perameisensäure, Peressigsäure, Perbenzoesäure oder m-Chlorperbenzoesäure ; Peroidate, wie Natrium- oder Kaliumperiodat ; Permanganate, wie Natrium- oder Kaliumpermanganat ; tert.-Butylhypochlorit oder Jodbenzoldichlorid.

Als Lösungsmittel können benutzt werden : Wasser ; Carbonsäuren wie Ameisen-, Essig- oder Propionsäure ; halogenierte Kohlenwasserstoffe wird Dichlormethan, Chloroform, 1,2-Dichlorethan, 1,1,1-Trichlorethan oder 1,1,2,2-Tetrachlorethan.

Die Oxidation wird z. B. bei Temperaturen zwischen — 20 und + 120 °C, vorzugsweise zwischen — 10 und 80 °C, ausgeführt. Je nach Anzahl der eingesetzten Äquivalente Oxidationsmittel je Äquivalent der Verbindung IV können die entsprechenden Sulfoxide (I, m = 1) oder die Sulfone (I, m = 2) erhalten werden.

Die Herstellung der erforderlichen 4,5-dihalogensubstituierten Pyridazinone II ist bekannt (vgl. K. Dury, Angew. Chem. 77, 282 bis 290 (1965)), ebenso die der benötigten Mercaptane (vgl. J. L. Wardell

in S. Patai, « The Chemistry of the Thiol Group » Bd. 1, S. 179 bis 211, Wiley & Sons New York 1974).

Die nachfolgenden Beispiele dienen zur Erläuterung der Erfindung, ohne diese einzuschränken.

Vorschrift 1

Herstellung von 5-Benzylthio-4-chlor-2-phenyl-pyridazin-3(2H)-on mit Natriummethylat als Base.

Man legt 5,4 g (100 mmol) Natriummethylat in 250 ml trockenem Methanol vor, kühlt auf 0 °C ab, tropft langsam 12,4 g (100 mmol) Benzylmercaptan zu und gibt anschließend bei der gleichen Temperatur 24,1 g (100 mmol) 4,5-Dichlor-2-phenyl-pyridazin-3(2H)-on zu. Danach läßt man die Temperatur langsam auf Raumtemperatur (20 °C) kommen und rührt 24 h nach, zuletzt 5 h bei 60 °C. Die Reaktionslösung wird eingeengt, der Rückstand mit Wasser versetzt, mit Salzsäure auf pH 3 gestellt und mit Dichlormethan extrahiert. Nach Waschen der organ. Phase mit verd. $NaHCO_3$-Lösung wird diese über $MgSO_4$ getrocknet und eingeengt. Abziehen des Solvens ergibt 29,1 g Rohmaterial, das man aus Ethanol umkristallisiert. Ein darin unlöslicher Rückstand (5,0 g) wird abfiltriert ; er besitzt nach Trocknen einen Schmp. von 158 bis 160 °C (Lit. 163 bis 164 °C ; vgl. R.N. Castle und K. Kaji, J. Heterocyclic Chem. 2, 463 bis 472 (1965)) und ist identisch mit 4,5-Bis(benzylthio)-2-phenyl-pyridazin-3(2H)-on (VIII, $R^1 = C_6H_5$, $R^2 = CH_2C_6H_5$). Das aus dem Filtrat auskristallisierende Material (21,2 g) besitzt doppelten Schmp. (115 bis 119 °C ; 135 bis 140 °C), weist im Dünnschichtchromatogramm zwei eng benachbarte Flecke auf und besteht aus 5-Benzylthio-4-chlor-pyridazin-3(2H)-on (IV, $R^1 = R^2 = C_6H_5$, X = Cl, n = 1) sowie dem stellungsisomeren 4-Benzylthio-5-chlor-2-phenyl-pyridazin-3(2H)-on (VII, $R^1 = C_6H_5$, $R^2 = CH_2C_6H_5$, X = Cl) im ungefähren Verhältnis 1 : 1 (NMR-Analyse). Durch säulenchromatographische Trennung ist hieraus das 5-Benzylthio-Derivat erhältlicht.

Vorschrift 2

Herstellung von 5-Benzylthio-4-chlor-2-phenylpyridazin-3(2H)-on mit Triethylamin als Base

24,1 g (100 ml) 4,5-Dichlor-2-phenyl-pyridazin-3(2H)-on werden bei — 20 °C in 150 ml Isopropanol suspendiert und eine gekühlte Mischung aus 12,4 g (100 mmol) Benzylmercaptan, 10,1 g (100 mmol) Triethylamin und 150 ml Isopropanol zugetropft. Anschließend wird 2 h bei — 10 °C nachgerührt, über Nacht bei Raumtemperatur sowie danach 2,5 h bei 40 °C. Nach Abkühlen saugt man den entstandenen Niederschlag ab, wäscht mit Isopropanol und Wasser nach und löst aus Toluol um. Das erhaltene Material (19,5 g, 59 %) ist dünnschichtchromatographisch einheitlich und schmilzt bei 139 bis 140 °C.

Vorschrift 3

Herstellung von 5-(4-Chlor-benzylthio)-4-chlor-2-phenylpyridazin-3(2H)-on

Die wie in Vorschrift 2 beschrieben vorgenommene Umsetzung von 15,8 g (100 mmol) 4-Chlorbenzylmercaptan mit 24,1 g (100 mmol) 4,5-Dichlor-2-phenyl-pyridazin-3(2H)-on und 10,1 g (100 mmol) Triethylamin liefert 30,5 g (84 %) dünnschichtchromatographisch einheitliches Material mit Schmp. 154 bis 155 °C.

Vorschrift 4

Herstellung von 5-Benzylthio-4-chlor-2-methyl-pyridazin-3(2H)-on

35,8 g (200 mmol) 4,5-Dichlor-2-methyl-pyridazin-3(2H)-on werden in 300 ml Isopropanol suspendiert und auf — 20 °C abgekühlt. Bei dieser Temperatur wird eine Lösung von 24,8 g (200 mmol) Benzylmercaptan und 20,2 g (200 mmol) Triethylamin in 200 ml Isopropanol zugetropft, danach 1 h bei — 10 °C sowie über Nacht bei Raumtemperatur nachgerührt. Den entstandenen Niederschlag saugt man ab, wäscht mit Isopropanol und Wasser nach und trocknet im Vakuum. Ausb. 31,7 g (60 %), Schmp. 102 bis 104 °C.

Vorschrift 5

Herstellung von 5-Benzylthio-4-brom-2-methyl-pyridazin-3(2H)-on

Wie in Vorschrift 4 angegeben bringt man 40,2 g (150 mmol) 4,5-Dibrom-2-methyl-pyridazin-3(2H)-on mit 18,6 g (150 mmol) Benzylmercaptan und 15,1 g (150 mmol) Triethylamin in insgesamt 400 ml Isopropanol zur Reaktion. Ausbeute 20,0 g (43 %), Schmp. 118 bis 121 °C.

Beispiel 1

Herstellung von 5-Benzylsulfonyl-4-chlor-2-phenyl-pyridazin-3(2H)-on

9,8 g (30 mmol) 5-Benzylthio-4-chlor-2-phenyl-pyridazin-3(2H)-on aus Vorschrift 2 wurden in 100 ml Eisessig bei 60 °C gelöst und 25 ml 30 proz. Wasserstoffperoxid zugetropft. Anschließend hielt man 3 h bei 65 °C, ließ abkühlen, saugte den entstandenen Niederschlag ab, wusch ihn mit Wasser peroxidfrei und trocknette im Vakuum. Ausbeute 7,9 g (73 %), Schmp. 154 bis 156 °C (Wirkstoff Nr. 61 b)

Beispiel 2

Herstellung von 5-Benzylsulfinyl-4-chlor-2-methyl-pyridazin-3(2H)-on

16,0 g (60,0 mmol) 5-Benzylthio-4-chlor-2-methyl-pyridazin-3(2H)-on aus Vorschrift 4 wurden in 200 ml Dichlormethan gelöst, auf — 20 °C abgekühlt und eine Lösung von 11,4 g (66,1 mmol) m-Chlorperbenzoesäure in Dichlormethan zugetropft. Man ließ 1 h bei — 10 °C, danach über Nacht bei Raumtemperatur nachrühren. Die Reaktionslösung wurde dreimal mit gesättigter $NaHCO_3$-Lösung und einmal mit Wasser gewaschen, getrocknet und eingeengt. Der Rückstand wurde mit Diethylether angerieben und abgesaugt. Ausbeute 13,1 g (77 %), Schmp. 129 bis 131 °C (Wirkstoff Nr. 2 a).

Nach den genannten Methoden lassen sich folgende Pyridazinon-Derivate herstellen :

(Siehe Tabellen Seite 6 ff.)

Die neuen Wirkstoffe zeigen eine starke fungitoxische Wirksamkeit gegen phytopathogene Pilze. Sie dienen insbesondere zur Verhütung und Heilung von Pflanzenkrankheiten, die durch Mikroorganismen verursacht werden, wie z. B. Phytophthora infestans, Botrytis cinerea, Plasmopara viticola, Monilia fructigena, Alternaria solani, Sclerotinia sclerotiorum, Pyricularia oryzae, Pellicularia filamentosa, Sclerotinia cinerea.

Die Wirkstoffe können gleichzeitig das Wachstum von zwei oder mehr der genannten Pilze unterdrücken und besitzen eine hohe Pflanzenverträglichkeit. Ein Teil der Wirkstoffe zeigt kurative Eigenschaften, d. h. die Anwendung der Mittel kann noch nach erfolgter Infektion der Pflanzen durch die Krankheitserreger vorgenommen werden, um einen sicheren Bekämpfungserfolg zu erzielen.

Die fungiziden Mittel enthalten 0,1 bis 95 % (Gewichtsprozent) Wirkstoff, vorzugsweise 0,5 bis 90 %. Die Aufwandmengen liegen je nach Art des gewünschten Effektes zwischen 0,1 bis 5 kg Wirkstoff je ha.

Die Wirkstoffe können auch zusammen mit anderen Wirkstoffen, z. B. Herbiziden, Insektiziden, Wachstumsregulatoren und anderen Fungiziden, oder auch mit Düngemitteln vermischt ausgebracht werden. In vielen Fällen erhält man bei der Mischung mit Fungiziden auch eine Vergrößerung des fungiziden Wirkungsspektrums ; bei einer Anzahl dieser Fungizidmischungen treten auch synergistische Effekte auf, d. h. die fungizide Wirksamkeit des Kombinationsproduktes ist größer als die der addierten Wirksamkeiten der Einzelkomponenten.

Fungizide, die mit den neuen Verbindungen kombiniert werden können, sind beispielsweise :

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid
Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,

heterocyclische Strukturen, wie
2-Heptadecyl-2-imidazolin-acetat
2,4-Dichlor-6-(o-chloranilino)-s-triazin
0,0-Diethyl-phthalimidophosphonothioat
5-Amino-1-(bis-(dimethylamino)-phosphinyl)-3-phenyl-1,2,4- triazol)
2,3-Dicyano-1,4-dithiaanthrachinon
2-Thio-1,3-dithio-(4,5,6)-chinoxalin

Tabelle 1

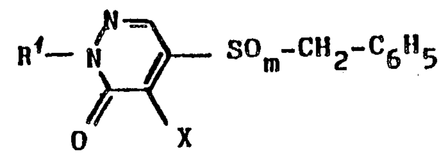

| Nr. | $R^1$ | X | m = 0 (Vorprodukt) | m = 1 a | m = 2 b |
|---|---|---|---|---|---|
| 1 | H | Cl | 183-187 | 212 (Zers.) | 236 (Zers.) |
| 2 | $CH_3$ | Cl | 102-104 | 129-131 | 186-188 |
| 3 | $CH_3$ | Br | 118-121 | 129-132 | 155-157 |
| 4 | $C_2H_5$ | Cl | 114-116 | | 117-119 |
| 5 | n-$C_3H_7$ | Cl | | | |
| 6 | i-$C_3H_7$ | Cl | 106-108 | 75- 77 | 130-132 |
| 7 | n-$C_4H_9$ | Cl | 88- 90 | | 82- 84 |
| 8 | i-$C_4H_9$ | Cl | | | |
| 9 | t-$C_4H_9$ | Cl | 72- 75 | | 142-146 |
| 10 | $(H_3C)_3C-CH_2$ | Cl | 108-110 | 115-118 | 107-109 |
| 11 | $(C_2H_5)(H_3C)CH$ | Cl | 107-109 | | 78- 81 |
| 12 | $(H_3C)_2CHCH_2(H_3C)CH$ | Cl | 88- 90 | 66- 69 | 70- 72 |
| 13 | cyclo-$C_6H_{11}$ | Cl | 167-170 | 156-159 | 175-179 |
| 14 | $HOCH_2$ | Cl | 164-166 | | |
| 15 | $HOCH_2$ | Br | | | |
| 16 | $HO_2C$ | Cl | | | 238 (Zers.) |
| 17 | $HO_2C$ | Br | | | |
| 18 | $H_3CCOOCH_2$ | Cl | 120-122 | | 112-114 |

Tabelle 1 (Fortsetzung)

| Nr. | $R^1$ | X | m = 0 (Vorprodukt) | m = 1 a | m = 2 b |
|---|---|---|---|---|---|
| 19 | $H_3CO(CH_2)_2OCH_2$ | Cl | | | |
| 20 | $H_5C_6O(CH_2)_2OCH_2$ | Cl | | | |
| 21 | $H_5C_6O(CH_2)_2O(CH_2)_2OCH_2$ | Cl | | | |
| 22 | $ClCH_2$ | Cl | | | |
| 23 | $F_3CCH_2$ | Cl | 113-115 | | 139-141 |
| 24 | $Cl_3CCH_2$ | Cl | | | |
| 25 | $2-Cl-C_6H_4$ | Cl | | | |
| 26 | $3-Cl-C_6H_4$ | Cl | 160-162 | 110-116 | 165-167 |
| 27 | $4-Cl-C_6H_4$ | Cl | | | |
| 28 | $3,5-Cl_2-C_6H_3$ | Cl | 129-131 | 151-153 | 181-184 |
| 29 | $2,4,6-Cl_3-C_6H_2$ | Cl | 171-174 | 149-152 | 177-179 |
| 30 | $3-Br-C_6H_4$ | Cl | | | |
| 31 | $4-Br-C_6H_4$ | Cl | 182-184 | | 171-174 |
| 32 | $3-(F_3C)-C_6H_4$ | Cl | 133-135 | 116-117 | 179-181 |
| 33 | $3-(F_3C)-C_6H_4$ | Br | 165-167 | 89- 93 | 164-166 |
| 34 | $4-(F_3C)-C_6H_4$ | Cl | | | |
| 35 | $4-(F_3C)-C_6H_4$ | Br | | | |
| 36 | $3-(F_3CO)-C_6H_4$ | Cl | | | |
| 37 | $4-(F_3CO)-C_6H_4$ | Cl | | | |
| 38 | $3-(F_2HCO)-C_6H_4$ | Cl | | | |
| 39 | $3-(F_2HCO)-C_6H_4$ | Br | 133-135 | | 118-122 |

Tabelle 1 (Fortsetzung)

| Nr. | R | X | m = 0 (Vorprodukt) | m = 1 a | m = 2 b |
|---|---|---|---|---|---|
| 40 | 4-($F_2CHCF_2O$)-$C_6H_4$ | Cl | | | |
| 41 | 4-($F_2CHCF_2O$)-$C_6H_4$ | Br | 154-156 | 172-174 | 207-211 |
| 42 | 3-($H_3CO$)-$C_6H_4$ | Cl | | | |
| 43 | 4-($H_3CO$)-$C_6H_4$ | Cl | | | |
| 44 | 4-($H_3CS$)-$C_6H_4$ | Cl | | | |
| 45 | 4-($H_3CSO_2$)-$C_6H_4$ | Cl | | | |
| 46 | 2-($H_3C$)-$C_6H_4$ | Cl | | | |
| 47 | 3-($H_3C$)-$C_6H_4$ | Cl | | | |
| 48 | 4-($H_3C$)-$C_6H_4$ | Cl | | | |
| 49 | 4-($H_3COOC$)-$C_6H_4$ | Cl | | | |
| 50 | 4-$(H_3C)_2NOC$-$C_6H_4$ | Cl | | | |
| 51 | 2-($O_2N$)-$C_6H_4$ | Cl | | | |
| 52 | 3-($O_2N$)-$C_6H_4$ | Cl | | | |
| 53 | 4-($O_2N$)-$C_6H_4$ | Cl | 208-212 | | 180-183 |
| 54 | 4-($O_2N$)-$C_6H_4$ | Br | | | |
| 55 | 2,4-$(O_2N)_2$-$C_6H_3$ | Cl | 172-175 | | 169-172 |
| 56 | 2,4-$(O_2N)_2$-$C_6H_3$ | Br | | | |
| 57 | 2-(NC)-$C_6H_4$ | Cl | | | |
| 58 | 3-(NC)-$C_6H_4$ | Cl | | | |
| 59 | 4-(NC)-$C_6H_4$ | Cl | | | |
| 60 | 4-(NCS)-$C_6H_4$ | Cl | | | |

Tabelle 2

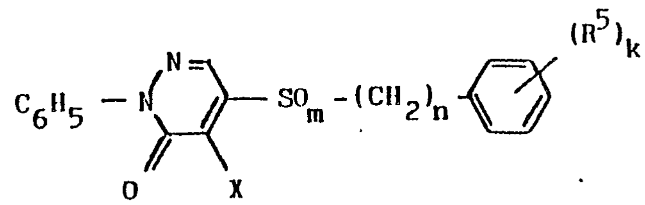

| Nr. | $(R^5)_k$ | n | X | Schmp. (°C) m = 0 (Vorprodukt) | m = 1 a | m = 2 b |
|---|---|---|---|---|---|---|
| 61 | H | 1 | Cl | 139–140 | | 154–156 |
| 62 | H | 1 | Br | 128–131 | 104–106 | 182–183 |
| 63 | H | 2 | Cl | 94– 96 | | 130–132 |
| 64 | H | 3 | Cl | 94– 96 | | 127–130 |
| 65 | 2-($H_3C$) | 1 | Cl | 164–166 | | 158–160 |
| 66 | 3-($H_3C$) | 1 | Cl | 128–130 | | 148–150 |
| 67 | 4-($H_3C$) | 1 | Cl | 135–137 | 133–135 | 176–178 |
| 68 | 4-($H_3C$) | 1 | Br | | | |
| 69 | 3-(n-$C_3H_7$) | 1 | Cl | | | |
| 70 | 4-(n-$C_3H_7$) | 1 | Cl | | | |
| 71 | 3-(i-$C_3H_7$) | 1 | Cl | | | |
| 72 | 4-(i-$C_3H_7$) | 1 | Cl | 140–142 | 103–106 | 170–173 |
| 73 | 4-(n-$C_4H_9$) | 1 | Cl | | | |

Tabelle 2 (Fortsetzung)

| Nr. | $(R^5)_k$ | n | X | Schmp. (°C) m = 0 (Vorprodukt) | m = 1 a | m = 2 b |
|---|---|---|---|---|---|---|
| 74 | 4-(i-$C_4H_9$) | 1 | Cl | | | |
| 75 | 4-(t-$C_4H_9$) | 1 | Cl | 184-186 | 157-160 | 178-180 |
| 76 | 2-F | 1 | Cl | | | |
| 77 | 3-F | 1 | Cl | 140-143 | | 189-190 |
| 78 | 4-F | 1 | Cl | 140-143 | 166-168 | 207-210 |
| 79 | 2-Cl | 1 | Cl | 162-164 | | 173-176 |
| 80 | 3-Cl | 1 | Cl | 148-151 | | 176-178 |
| 81 | 4-Cl | 1 | Cl | 154-155 | | 222-225 |
| 82 | 3,4-$Cl_2$ | 1 | Cl | 170-172 | 193-196 | 218-220 (Zers.) |
| 83 | 3,5-$Cl_2$ | 1 | Cl | | | |
| 84 | 2,4-$Cl_2$ | 1 | Cl | | | |
| 85 | 2,6-$Cl_2$ | 1 | Cl | | | |
| 86 | 3-Br | 1 | Cl | | | |
| 87 | 4-Br | 1 | Cl | | | |
| 88 | 3-$H_3CO$ | 1 | Cl | 132-134 | | 162-163 |
| 89 | 4-$H_3CO$ | 1 | Cl | 212-214 | | 166-168 |
| 90 | 3-CN | 1 | Cl | 183-186 | | 187-189 |
| 91 | 4-CN | 1 | Cl | 190-192 | | 222 (Zers.) |
| 92 | 2-$NO_2$ | 1 | Cl | | | |
| 93 | 3-$NO_2$ | 1 | Cl | 176-177 | | 186-189 |
| 94 | 4-$NO_2$ | 1 | Cl | 175-177 | | mehr als 240 |
| 95 | 3,5-$(NO_2)_2$ | 1 | Cl | | | |
| 96 | 4-$H_3CS$ | 1 | Cl | | | |
| 97 | 4-$H_3CSO_2$ | 1 | Cl | | | |
| 98 | 4-$(H_3C)_2N(OC)$ | 1 | Cl | | | |
| 99 | 4-NCS- | 1 | Cl | | | |

Tabelle 3

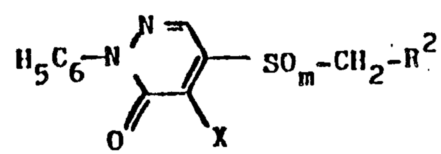

| Nr. | $R^2$ | X | m = 0 (Vorprodukt) | m = 1 a | m = 2 b |
|---|---|---|---|---|---|
| 100 | $H_2C=CH$ | Cl | | | |
| 101 | $H_2C=CH$ | Br | | | |
| 102 | $H_2C=C(CH_3)$ | Cl | | | |
| 103 | $H_2C=C(CH_3)$ | Br | | | |

Tabelle 4

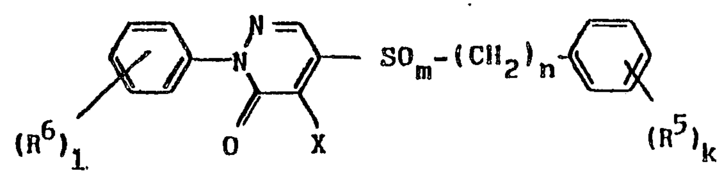

| Nr. | $(R^6)_l$ | $(R^5)_k$ | n | X | Schmp. (°C) m = 0 (Vorprodukt) | m = 1 a | m = 2 b |
|---|---|---|---|---|---|---|---|
| 104 | H | 3-F | 1 | Cl | | | |
| 105 | H | 4-F | 1 | Cl | | | |
| 106 | $H_3C$ | 3-F | 1 | Cl | | | |
| 107 | $H_3C$ | 4-F | 1 | Cl | | | |
| 108 | $H_3C$ | 4-F | 2 | Cl | | | |
| 109 | $H_3C$ | 4-F | 1 | Br | | | |
| 110 | $H_3C$ | 3-($H_3CO$) | 1 | Cl | | | |
| 111 | $H_3C$ | 4-($H_3CO$) | 1 | Cl | | | |
| 112 | $H_3C$ | 4-($H_3CO$) | 1 | Br | | | |
| 113 | $H_3C$ | 3-$H_3C$ | 1 | Cl | | | |
| 114 | $H_3C$ | 4-$H_3C$ | 1 | Cl | | | |
| 115 | $H_3C$ | 3,4-$(H_3C)_2$ | 1 | Cl | | | |
| 116 | $C_6H_5$ | 3-F | 1 | Cl | | | |

Tabelle 4 (Fortsetzung)

| Nr. | $(R^6)_{k1}$ | $(R^5)_k$ | n | X | Schmp. (°C) m = 0 (Vorprodukt) | m = 1 a | m = 2 b |
|---|---|---|---|---|---|---|---|
| 117 | $C_6H_5$ | 4-F | 1 | Cl | | | |
| 118 | $C_6H_5$ | 4-F | 2 | Cl | | | |
| 119 | $C_6H_5$ | 4-F | 1 | Br | | | |
| 120 | $4-(O_2N)-C_6H_4$ | 3-F | 1 | Cl | | | |
| 121 | $4-(O_2N)-C_6H_4$ | 4-F | 1 | Cl | | | |
| 122 | $HOCH_2$ | 3-F | 1 | Cl | | | |
| 123 | $HOCH_2$ | 4-F | 1 | Cl | | | |
| 124 | $H_5C_6O(CH_2)_2OCH_2$ | 3-F | 1 | Cl | | | |
| 125 | $H_5C_6O(CH_2)_2OCH_2$ | 4-F | 1 | Cl | | | |

Tabelle 5

| Nr. | $R^1$ | X | m | n | $R^2$ | Schmp. (°C) |
|---|---|---|---|---|---|---|
| 126 | $t-C_4H_9$ | Cl | 2 | 3 | $C_6H_5$ | 103 - 104 |

1-(Butylcarbamoyl)-2-benzimidazol-carbaminsäuremethylester
2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethyl-phthalimid
N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Jod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1-H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
α-(2-Chlorphenyl)-α-(4-chlorphenyl)-5-pyrimidin-methanol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene Fungizide, wie
Dodecylguanidinacetat
3-(3-(3,5-Dimethyl-2-oxycyclohexyl)-2-hydroxyethyl)-glutaramid
Hexachlorbenzol
DL-Methyl-N-(2,6-dimethyl-phenyl)-N-furoyl(2)-alaninat,
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-2,4-dioxo-1,3-oxazolidin
3-(3,5-Dichlorphenyl)-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid.

Die neuen Wirkstoffe werden beispielsweise in Form von direkt versprühbaren Lösungen, Suspensionen, Emulsionen, auch in Form von hochprozentigen wäßrigen, öligen oder sonstigen Dispersionen, Pasten, Stäubemitteln, Streumitteln, Granulaten durch Versprühen, Verstäuben, Verstreuen, Verstreichen oder Gießen ausgebracht. Die Anwendungsformen richten sich ganz nach den Verwendungszwecken ; sie sollten in der Regel möglichst eine feine Verteilung der neuen Wirkstoffe gewährleisten.

Zur Herstellung von direkt oder nach Emulgieren in Wasser verwendbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische oder aromatische Kohlenwasserstoffe, zum Beispiel Benzol, Toluol, Xylol, Paraffin, Tetrahydronaphthalin, alkylierte Naphthaline oder deren Derivate z. B. Methanol, Ethanol,

Propanol, Butanol, Chloroform, Tetrachlorkohlenstoff, Cyclohexanol, Cyclohexanon, Chlorbenzol, Isophoron usw., stark polare Lösungsmittel, wie z. B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäure, Alkylarylsulfonate, Alkylsulfate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalin-derivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Beispiele für solche Pflanzenschutzmittel-Zubereitungen sind :

I. Man vermischt 90 Gewichtsteile der Verbindung des Beispiels 1 mit 100 Gewichtsteilen N-Methylpyrrolidon und erhält eine Lösung, die zur Anwendung in Form kleinster Trofpfen geeignet ist.

II. 20 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Doedecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der nach Beispiel 1 erhältlichen Verbindung werden in einer Mischung gelöst, die aus 30 Gewichtsteilen Cyclohexanon, 30 Gewichtsteilen Isobutanol, 20 Gewichtsteilen des Anlagerungsproduktes von 40 ml Ethylenoxid an 1 Mol Ricinusöl besteht. Durch Eingießen und Verrühren der Lösung in Wasser erhält man eine wäßrige Dispersion.

IV. 20 Gewichtsteile der nach Beispiel 2 erhältlichen Verbindung werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und Verrühren der Lösung in Wasser, erhält man eine wäßrige Dispersion.

V. 20 Gewichtsteile der nach Beispiel 1 erhältlichen Verbindung werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in Wasser erhält man eine Spritzbrühe.

VI. 5 Gewichtsteile der nach Beispiel 2 erhältlichen Verbindung werden mit 95 Gewichtsteilen feinteiligem Kaolin innig vermischt. Man erhält auf diese Weise ein Stäubemittel, das 5 Gew.-% des Wirkstoffs enthält.

VII. 30 Gewichtsteile der nach Beispiel 1 erhältlichen Verbindung werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese eine Aufbereitung des Wirkstoffs mit guter Haftfähigkeit.

VIII. 40 Gewichtsteile der nach Beispiel 2 erhältlichen Verbindung werden mit 30 Teilen Natriumsalz eines Phenolsulfonsäure-Harnstoff-Formaldehyd-Kondensates, 2 Teilen Kieselgel und 48 Teilen Wasser innig vermischt. Man erhält eine stabile wäßrige Dispersion.

IX. 20 Teile der nach Beispiel 1 erhältlichen Verbindung mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt.

Man erhält eine stabile ölige Dispersion.

Die folgenden Versuche belegen die biologische Wirkung der neuen Verbindungen. Vergleichsmittel (Vglm.) ist stets der bekannte, insbesondere zur Bekämpfung von Botrytis geeignete Wirkstoff N-Trichlormethylthio-tetra-hydrophthalimid (Chem. Week, June 21, 1972, Seite 46).

Beispiel 1

Wirksamkeit gegen Botrytis cinerea an Paprika

Paprikasämlinge der Sorte « Neusiedler Ideal Elite » werden, nachdem sich 4 bis 5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24 °C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß beispielsweise bei der Anwendung als 0,05 %ige Wirkstoffbrühe die Wirkstoffe 2a, 2b, 3b, 4b, 6b, 9b, 10b, 11b, 16b, 61b, 62b und 63b eine bessere fungizide Wirkung (beispielsweise 97 %) zeigen als das Vergleichsmittel (70 %).

Beispiel 2

Wirksamkeit gegen Phytophthora infestans an Tomaten

Blätter von Topfpflanzen der Sorte « Große Fleischtomate » werden mit wäßriger Spritzbrühe, die 80 % Wirkstoff und 20 % Emulgiermittel in der Trockensubstanz enthält, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18 °C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

Das Ergebnis des Versuches zeigt, daß beispielsweise bei der Anwendung als 0,025 %ige Wirkstoffbrühe die Wirkstoffe 1b, 2b, 3b, 4b, 6b, 9b, 10b, 11b, 18b, 61b, 62b, 63b, 67b und 72b eine bessere fungizide Wirkung (beispielsweise 97 %) zeigen als das Vergleichsmittel (70 %).

Beispiel 3

Wirksamkeit gegen Plasmopara viticola

Blätter von Topfreben der Sorte « Müller-Thurgau » werden mit wäßriger Spritzbrühe, die 80 % Wirkstoff und 20 % Emulgiermittel in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 Stunden in einer wasserdampfgesättigten Kammer bei 24 °C und anschließend für 8 Tage in einem Gewächshaus mit Temperaturen zwischen 20 und 30 °C aufgestellt. Nach dieser Zeit werden die Pflanzen zur Beschleunigung des Sporangienträgerausbruches abermals für 16 Stunden in der feuchten Kammer aufgestellt.

Dann erfolgt die Beurteilung des Ausmaßes des Pilzausbruches auf den Blattunterseiten.

Das Ergebnis des Versuches zeigt, daß beispielsweise die Wirkstoffe 1b, 2b, 3b, 4b, 6a, 6b, 7b, 9b, 10b, 11b, 12b, 13b, 16b, 18b, 23b, 33b, 62b, 64b, 67b, 78b, 89b und 91b eine gute fungizide Wirkung (beispielsweise 90 %) haben.

**Patentansprüche** (für die Vertragsstaaten : BE, CH, DE, FR, GB, IT, LI, NL, SE)

1. Pyridazinon-Derivat der Formel I

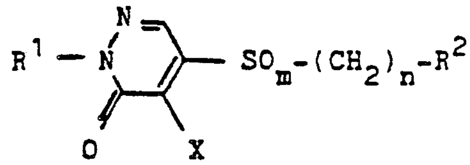

(I)

worin

$R^1$ Wasserstoff ; $C_1$-$C_8$-Alkyl, ggf. ein- oder mehrfach durch Halogen substituiert ; $C_3$-$C_7$-Cycloalkyl ; Phenyl, ggf. ein- oder mehrfach, gleich oder verschieden substituiert durch Halogen, $C_1$-$C_4$-Alkyl, Mono-, Di- oder Trihalogen-$C_1$-$C_3$-Alkyl, Mono-, Di- oder Trihalogen-$C_1$-$C_3$-Alkoxy, $C_1$-$C_4$-Alkoxy, $C_1$-$C_4$-Alkylthio, $C_1$-$C_4$-Alkyl-sulfinyl, $C_1$-$C_4$-Alkylsulfonyl, $C_1$-$C_4$-Alkoxycarbonyl, Mono- oder Di-($C_1$-$C_4$-Alkyl)-carbamoyl, Cyan, Thiocyan oder Nitro ; Carboxyl ; Hydroxymethyl ; $—CH_2O—COR^3$, wobei $R_3$ $C_1$-$C_6$-Alkyl bedeutet ; $—CH_2O[CH_2CH_2O]_pR^4$ wobei $R^4$ $C_1$-$C_4$-Alkyl oder Phenyl und p 1, 2 oder 3 bedeutet ;

$R^2$ Phenyl, ggf. ein- oder mehrfach, gleich oder verschieden substituiert durch Halogen, $C_1$-$C_4$-Alkyl (ggf. ein- oder mehrfach mit Halogen substituiert), $C_1$-$C_4$-Alkoxy (ggf. ein- oder mehrfach mit Halogen substituiert), $C_1$-$C_4$-Alkylthio, $C_1$-$C_4$-Alkylsulfinyl, $C_1$-$C_4$-Alkylsulfonyl, Mono- oder Di-($C_1$-$C_4$-Alkyl)-carbamoyl, Cyan, Thiocyan oder Nitro ; Vinyl oder 2-Propenyl ;

X Halogen

m 1 oder 2 und

n 1, 2, 3 oder 4

bedeuten.

2. 5-Benzylsulfonyl-4-chlor-2-phenyl-pyridazin-3(2H)-on.

3. 5-Benzylsulfonyl-4-brom-2-phenyl-pyridazin-3(2H)-on

4. 5-Benzylsulfonyl-4-chlor-2-methyl-pyridazin-3(2H)-on.

5. 5-Benzylsulfonyl-4-brom-2-methyl-pyridazin-3(2H)-on.

6. Verfahren zur Herstellung von Pyridazinon-Derivaten der Formel I gemäß Anspruch 1, dadurch gekennzeichnet, daß man 4,5-Dihalogenpyridazin-3(2H)-one der Formel II

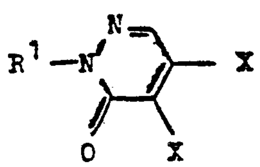

(II)

mit Mercaptanen der Formel III

$$R^2—(CH_2)_n—SH \quad (III),$$

worin $R^1$, $R^2$, X und n die in Anspruch 1 angegebene Bedeutung besitzen, in Anwesenheit einer Base umsetzt und die so erhaltenen 5-Thiopyridazin-3(2h)-on-Derivate der Formel IV

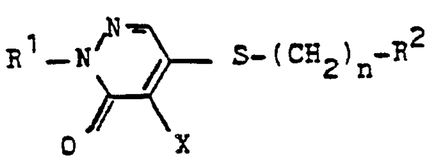

(IV)

zu Verbindungen der Formel I oxidiert.

7. Fungizid, enthaltend eine Verbindung der Formel I gemäß Anspruch 1.

8. Verfahren zur Bekämpfung von phytopathogemen Pilzen, dadurch gekennzeichnet, daß man eine fungizid wirksame Menge einer Verbindung der Formel I gemäß Anspruch 1 auf die Pilze oder durch Befall durch Pilze bedrohte Flächen, Pflanzen oder Saatgüter einwirken läßt.

9. Fungizid, enthaltend einen festen oder flüssigen Trägerstoff und eine Verbindung der Formel I gemäß Anspruch 1.

10. Verfahren gemäß Ansprüch 6, dadurch gekennzeichnet, daß man eine Verbindung der Formel II

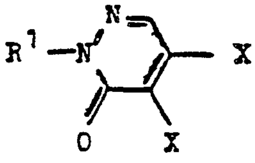

(II)

mit einem Mercaptan der Formel III

$$R^2—(CH_2)_n—SH \quad (III),$$

worin $R^1$, $R^2$, X und n die in Anspruch 1 angegebene Bedeutung haben, in Gegenwart eines Amins in einem nicht nucleophilen Lösungsmittel bei Temperaturen von —40 °C bis + 100 °C umsetzt.

**Patentansprüche** (für den Vertragsstaat AT)

1. Fungizid, enthaltend ein Pyridazon-Derivat der Formel I

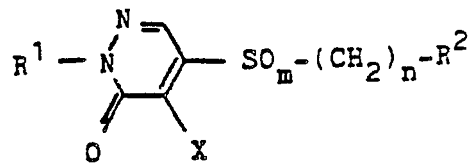

(I)

worin

$R^1$ Wasserstoff ; $C_1$-$C_8$-Alkyl, ggf. ein- oder mehrfach durch Halogen substituiert ; $C_3$-$C_7$-Cycloalkyl ; Phenyl, ggf. ein- oder mehrfach, gleich oder verschieden substituiert durch Halogen, $C_1$-$C_4$-Alkyl, Mono-, Di- oder Trihalogen-$C_1$-$C_3$-Alkyl, Mono-, Di- oder Trihalogen-$C_1$-$C_3$-Alkoxy, $C_1$-$C_4$-Alkoxy, C1-$C_4$-Alkylthio, $C_1$-$C_4$-Alkylsulfinyl, $C_1$-$C_4$-Alkylsulfonyl, $C_1$-$C_4$-Alkoxycarbonyl, Mono- oder Di-($C_1$-$C_4$-Alkyl)-carbamoyl, Cyan, Thiocyan oder Nitro ; Carboxyl ; Hydroxymethyl ; $—CH_2O—COR^3$, wobei $R^3$ $C_1$-$C_6$-Alkyl bedeutet ; $—CH_2O[CH_2CH_2O]_pR^4$ wobei $R^4$ $C_1$-$C_4$-Alkyl oder Phenyl und p 1, 2 oder 3 bedeutet ;

$R^2$ Phenyl, ggf. ein- oder mehrfach, gleich oder verschieden substituiert durch Halogen, $C_1$-$C_4$-Alkyl (ggf. ein- oder mehrfach mit Halogen substituiert), $C_1$-$C_4$-Alkoxy (ggf. ein- oder mehrfach mit Halogen substituiert), $C_1$-$C_4$-Alkylthio, $C_1$-$C_4$-Alkylsulfinyl, $C_1$-$C_4$-Alkylsulfonyl, Mono- oder Di-($C_1$-$C_4$-Alkyl)-carbamoyl, Cyan, Thiocyan oder Nitro ; Vinyl oder 2-Propenyl ;

X Halogen

m 1 oder 2 und

n 1, 2, 3 oder 4

bedeuten.

2. Fungizid, enthaltend einen festen oder flüssigen Trägerstoff und eine Verbindung der Formel I gemäß Anspruch 1.

3. Verfahren zur Bekämpfung von phytopatogenen Pilzen, dadurch gekennzeichnet, daß man eine fungizid wirksame Menge einer Verbindung der Formel I gemäß Anspruch 1 auf die Pilze oder durch Befall durch Pilze bedrohte Flächen, Pflanzen oder Saatgüter einwirken läßt.

4. Verfahren zur Herstellung von Pyridazinon-Derivaten der Formel I gemäß Anspruch 1, dadurch gekennzeichnet, daß man 4,5-Dihalogenpyridazin-3(2H)-one der Formel II

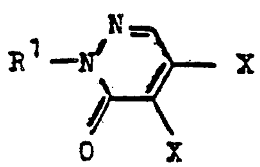

(II)

mit Mercaptanen der Formel III

$$R^2—(CH_2)_n—SH \quad (III),$$

worin $R^1$, $R^2$, X und n die in Anspruch 1 angegebene Bedeutung besitzen, in Anwesenheit einer Base umsetzt und die so erhaltenen 5-Thiopyridazin-3(2H)-on-Derivate der Formel IV

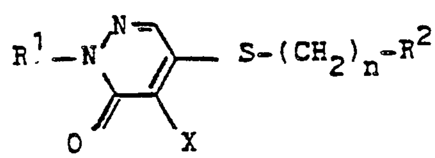

(IV)

zu Verbindungen der Formel I oxidiert.

5. Verfahren gemäß Anspruch 4

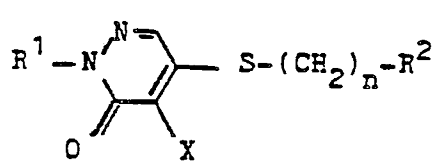

(IV)

dadurch gekennzeichnet, daß man eine Verbindung der Formel II

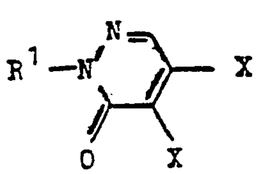

(II)

mit einem Mercaptan der Formel III

$$R^2—(CH_2)_n—SH \qquad (III),$$

worin $R^1$, $R^2$, X und n die in Anspruch 1 angegebene Bedeutung haben in Gegenwart eines Amins in einem nicht nucleophilen Lösungsmittel bei Temperaturen von —40 °C bis + 100 °C umsetzt.

**Claims** (for the Contracting States : BE, CH, DE, FR, GB, IT, LI, NL, SE)

1. A pyridazone derivative of the formula I

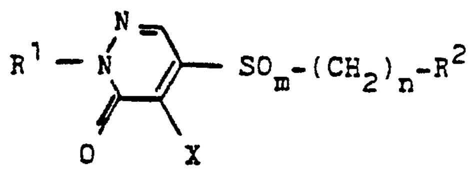

(I)

where

$R^1$ is hydrogen or $C_1$-$C_8$-alkyl which is unsubstituted or monosubstituted or polysubstituted by halogen, or is $C_3$-$C_7$-cycloalkyl, or is phenyl which is unsubstituted or monosubstituted or polysubstituted by halogen, $C_1$-$C_4$-alkyl, mono-, di- or trihalo-$C_1$-$C_3$-alkyl, mono-, di- or trihalo-$C_1$-$C_3$-alkoxy, $C_1$-$C_4$-alkoxy, $C_1$-$C_4$-alkylthio, $C_1$-$C_4$-alkylsulfinyl, $C_1$-$C_4$-alkylsulfonyl, $C_1$-$C_4$-alkoxycarbonyl, mono- or di-($C_1$-$C_4$-alkyl)-carbamyl, cyano, thiocyano or nitro, the substituents being identical or different in the case of polysubstitution, or is carboxyl, hydroxymethyl or $—CH_2—O—COR^3$, where $R^3$ is $C_1$-$C_6$-alkyl, or is $—CH_2O[CH_2CH_2O]_pR^4$, where $R^4$ is $C_1$-$C_4$-alkyl or phenyl, and p is 1, 2 or 3 ;

$R^2$ is phenyl which is unsubstituted or monosubstituted or polysubstituted by halogen, $C_1$-$C_4$-alkyl (unsubstituted or monosubstituted or polysubstituted by halogen), $C_1$-$C_4$-alkoxy (unsubstituted or monosubstituted or polysubstituted by halogen), $C_1$-$C_4$-alkylthio, $C_1$-$C_4$-alkylsulfinyl, $C_1$-$C_4$-alkylsulfonyl, mono- or di-($C_1$-$C_4$-alkyl)-carbamyl, cyano, thiocyano or nitro, the substituents being identical or different in the case of polysubstitution, or is vinyl or 2-propenyl ;

X is halogen ;

m is 1 or 2 ; and

n is 1, 2, 3 or 4.

2. 5-Benzylsulfonyl-4-chloro-2-phenylpyridazin-3(2H)-one.

3. 5-Benzylsulfonyl-4-bromo-2-phenylpyridazin-3(2H)-one.

4. 5-Benzylsulfonyl-4-chloro-2-methylpyridazin-3(2H)-one.

5. 5-Benzylsulfonyl-4-bromo-2-methylpyridazin-3(2H)-one.

6. A process for the preparation of a pyridazinone derivative of the formula I as claimed in claim 1, wherein a 4,5-dihalopyridazin-3(2H)-one of the formula II

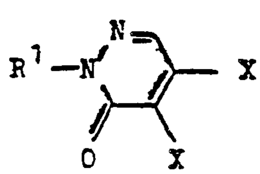

(II)

is reacted with a mercaptan of the formula III

$$R^2—(CH_2)_n—SH \qquad (III),$$

where $R^1$, $R^2$, X and n have the meanings given in claim 1, in the presence of a base, and the resulting 5-thiopyridazin-3(2H)-one derivative of the formula IV

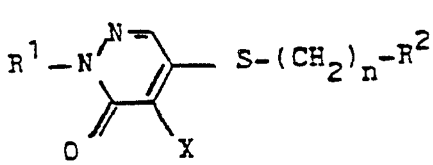

(IV)

is oxidized to give a compound of the formula I.

7. A fungicide containing a compound of the formula I as claimed in claim 1.

8. A process for combating phytopathogenic fungi, wherein a fungicidally effective amount of a compound of the formula I as claimed in claim 1 is allowed to act on the fungi or areas, plants or seed threatened by fungus attack.

9. A fungicide containing a solid or liquid carrier and a compound of the formula I as claimed in claim 1.

10. A process as claimed in claim 6, wherein a compound of the formula II

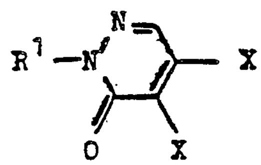

(II)

is reacted with a mercaptan of the formula III

$$R^2—(CH_2)_n—SH \quad (III),$$

where $R^1$, $R^2$, X and n have the meanings given in claim 1, in the presence of an amine and in a non-nucleophilic solvent at from —40 °C to + 100 °C.

**Claims** (for the Contracting State AT)

1. A fungicide containing a pyridazone derivative of the formula I

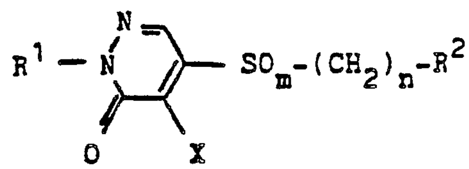

(I)

where

$R^1$ is hydrogen or $C_1$-$C_8$-alkyl which is unsubstituted or monosubstituted or polysubstituted by halogen, or is $C_3$-$C_7$-cycloalkyl, or is phenyl which is unsubstituted or monosubstituted or polysubstituted by halogen, $C_1$-$C_4$-alkyl, mono-, di- or trihalo-$C_1$-$C_3$-alkyl, mono-, di- or trihalo-$C_1$-$C_3$-alkoxy, $C_1$-$C_4$-alkoxy, $C_1$-$C_4$-alkylthio, $C_1$-$C_4$-alkylsulfinyl, $C_1$-$C_4$-alkylsulfonyl, $C_1$-$C_4$-alkoxycarbonyl, mono- or di-($C_1$-$C_4$-alkyl)-carbamyl, cyano, thiocyano or nitro, the substituents being identical or different in the case of polysubstitution, or is carboxyl, hydroxymethyl or $—CH_2O—COR^3$, where $R^3$ is $C_1$-$C_6$-alkyl, or is $—CH_2O[CH_2CH_2O]_pR^4$, where $R^4$ is $C_1$-$C_4$-alkyl or phenyl, and p is 1, 2 or 3 ;

$R^2$ is phenyl which is unsubstituted or monosubstituted or polysubstituted by halogen, $C_1$-$C_4$-alkyl (unsubstituted or monosubstituted or polysubstituted by halogen), $C_1$-$C_4$-alkoxy (unsubstituted or monosubstituted or polysubstituted by halogen), $C_1$-$C_4$-alkylthio, $C_1$-$C_4$-alkylsulfinyl, $C_1$-$C_4$-alkylsulfonyl, mono- or di-($C_1$-$C_4$-alkyl)-carbamyl, cyano, thiocyano or nitro, the substituents being identical or different in the case of polysubstitution, or is vinyl or 2-propenyl ;

X is halogen ;

m is 1 or 2 ; and

n is 1, 2, 3 or 4.

2. A fungicide containing a solid or liquid carrier and a compound of the formula I as claimed in claim 1.

3. A process for combating phytopathogenic fungi, wherein a fungicidally effective amount of a compound of the formula I as claimed in claim 1 is allowed to act on the fungi or areas, plants or seed threatened by fungus attack.

4. A process for the preparation of a pyridazinone derivative of the formula I as defined in claim 1, wherein a 4,5-dihalopyridazin-3(2H)-one of the formula II

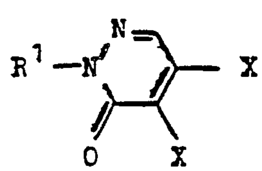

(II)

is reacted with a mercaptan of the formula III

$R^2—(CH_2)_n—SH$ (III),

where $R^1$, $R^2$, X and n have the meanings given in claim 1, in the presence of a base, and the resulting 5-thiopyridazin-3(2H)-one derivative of the formula IV

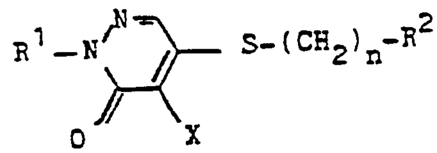

(IV)

is oxidized to give a compound of the formula I.

5. A process as claimed in claim 4, wherein a compound of the formula II

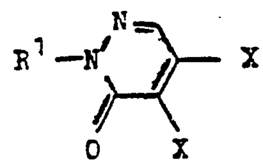

(II)

is reacted with a mercaptan of the formula III

$R^2—(CH_2)_n—SH$ (III),

where $R^1$, $R^2$, X and n have the meanings given in claim 1, in the presence of an amine and in a non-nucleophilic solvent at from —40 °C to + 100 °C.

**Revendications** (pour les Etats contractants : BE, CH, DE, FR, GB, IT, LI, NL, SE)

1. Dérivé de la pyridazinone de la formule I

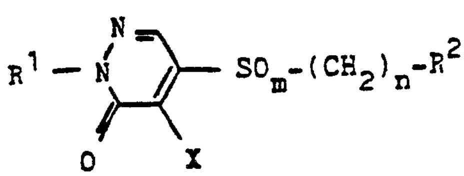

(I)

dans laquelle

$R^1$ peut désigner un atome d'hydrogène, un radical alkyle en $C_1$ à $C_8$ éventuellement mono- ou pluri-halo-substitué, un groupe cycloalkyle en $C_3$ à $C_7$, un groupe phényle pouvant porter un ou plusieurs substituants identiques ou différents choisis parmi les atomes d'halogène, les radicaux alkyle en $C_1$ à $C_4$, les radicaux alkyle en $C_1$ à $C_3$ mono- di- et tri-halogénés, les radicaux alcoxy en $C_1$ à $C_4$, les radicaux alcoxy en $C_1$ à $C_3$ mono-, di- et tri-halogénés et les groupes alkyl(en $C_1$ à $C_4$)-thio, alkyl(en $C_1$ à $C_4$)-sulfinyle, alkyl(en $C_1$ à $C_4$)-sulfonyle, alcoxy(en $C_1$ à $C_4$)-carbonyle, mono- et dialkyl (en $C_1$ à $C_4$)-carbamoyle, cyano, thiocyano et nitro ; un groupe carboxyle, hydroxyméthyle, $—CH_2O—COR^3$ où R = alkyle en $C_1$ à $C_6$, $—CH_2OCH_2CH_2O$ $pR^4$, où $R^4$ = alkyle en $C_1$ à $C_4$ ou phényle et p vaut 1, 2 ou 3 ;

$R^2$ peut désigner un groupe phényle pouvant porter un ou plusieurs substituants identiques ou différents choisis parmi les atomes d'halogène, les radicaux alkyle en $C_1$ à $C_4$ éventuellement mono- ou pluri-halo-substitués, les radicaux alcoxy en $C_1$ à $C_4$ éventuellement mono- ou pluri-halo-substitués et les groupes alkyl(en $C_1$ à $C_4$)-thio, alkyl(en $C_1$ à $C_4$)-sulfinyle, alkyl(en $C_1$ à $C_4$)-sulfonyle, mono- et di-alkyl(en $C_1$ à $C_4$)-carbamoyle, alcoxy(en $C_1$ à $C_4$)-carbonyle, cyano, thiocyano et nitro ; un groupe vinyle ou 2-propényle ;

X représente un atome d'halogène,

m vaut 1 ou 2 et

n vaut 1, 2, 3 ou 4.

2. La 5-benzylsulfonyl-4-chloro-2-phényl-pyridazine-3(2H)-one.

3. La 5-benzylsulfonyl-4-bromo-2-phényl-pyridazine-3(2H)-one.

4. la 5-benzyl-4-chloro-2-méthyl-pyridazine-3(2H)-one.

5. La 5-benzylsulfonyl-4-bromo-2-méthyl-pyridazine-3(2H)-one.

6. Procédé de préparation de dérivés de la pyridazinone de la formule I selon la revendiction 1, caractérisé en ce que l'on fait réagir une 4,5-dihalo-pyridazine-3 (2H)-one de la formule II

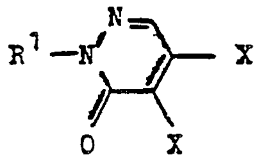

(II)

en présence d'une base avec un mercaptan de la formule III

$R^2—(CH_2)_n—SH$ (III),

dans lesquelles $R^1$, $R^2$, X et n possèdent les significations définies dans la revendication 1, puis l'on oxyde le dérivé de 5-thio-pyridazine-3(2H)-one obtenu de la formule IV

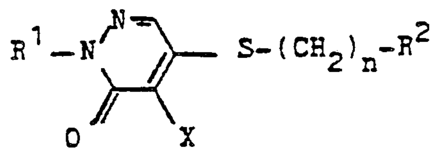

(IV)

en un composé de la formule I.

7. Composition fongicide, contenant un composé de la formule I selon la revendication 1.

8. Procédé de lutte contre les champignons phytopathogènes, caractérisé en ce que l'on traite les champignons ou les surfaces, plantes ou semences risquant d'être infestées par des champignons avec une quantité efficace du point de vue fongicide d'un composé de la formule I selon la revendication 1.

9. Composition fongicide, comprenant un composé de la formule I selon la revendication 1 dans un véhicule solide ou liquide.

10. Procédé suivant la revendication 6, caractérisé en ce que l'on fait réagir un composé de la formule II

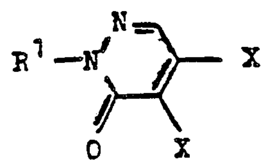

(II)

et un mercaptan de la formule III

$R^2—(CH_2)_n—SH$ (III),

dans lesquelles $R^1$, $R^2$, X et n possèdent la signification définie dans la revendication 1, à des températures comprises entre —40 et + 100 °C dans un solvant non nucléophile et en présence d'une amine.

**Revendications** (pour l'Etat contractant AT)

1. Composition fongicide, contenant un dérivé de la pyridazinone de la formule I

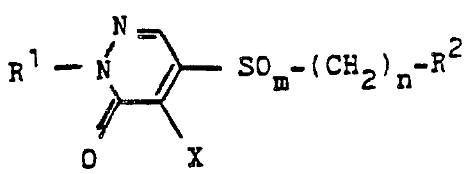

(I)

dans laquelle

$R^1$ peut désigner un atome d'hydrogène, un radical alkyle en $C_1$ à $C_8$ éventuellement mono- ou pluri-halo-substitué, un groupe cycloalkyle en $C_3$ à $C_7$, un groupe phényle pouvant porter un ou plusieurs substituants identiques ou différents choisis parmi les atomes d'halogène, les radicaux alkyle en $C_1$ à $C_4$, les radicaux alkyle en $C_1$ à $C_3$ mono-, di- et tri-halogénés, les radicaux alcoxy en $C_1$ à $C_4$, les radicaux alcoxy en $C_1$ à $C_3$ mono-, di- et tri-halogénés et les groupes alkyl(en $C_1$ à $C_4$)-thio, alkyl(en $C_1$ à $C_4$)-sulfinyle, alkyl(en $C_1$ à $C_4$)-sulfonyle, alcoxy(en $C_1$ à $C_4$)-carbonyle, mono- et dialkyl(en $C_1$ à $C_4$)-carbamoyle, cyano, thiocyano et nitro ; un groupe carboxyle, hydroxyméthyle, $—CH_2O—COR^3$ où R = alkyle en $C_1$ à $C_6$, $—CH_2OCH_2CH_2O_pR^4$, où $R^4$ = alkyle en $C_1$ à $C_4$ ou phényle et p vaut 1, 2 ou 3 ;

$R^2$ peut désigner un groupe phényle pouvant porter un ou plusieurs substituants identiques ou

différents choisis parmi les atomes d'halogène, les radicaux alkyle en $C_1$ à $C_4$ éventuellement mono- ou pluri-halo-substitués, les radicaux alcoxy en $C_1$ à $C_4$ éventuellement mono- ou pluri-halo-substitués et les groupes alkyl(en $C_1$ à $C_4$)-thio, alkyl(en $C_1$ à $C_4$)-sulfinyle, alkyl(en $C_1$ à $C_4$)-sulfonyle, mono- et di-alkyl(en $C_1$ à $C_4$)-carbamoyle, alcoxy(en $C_1$ à $C_4$)-carbonyle, cyano, thiocyano et nitro ; un groupe vinyle ou 2-propényle ;

X représente un atome d'halogène,

m vaut 1 ou 2 et

n vaut 1, 2, 3 ou 4.

2. Composition fongicide, comprenant un composé de la formule I selon la revendication 1 dans un véhicule solide ou liquide.

3. Procédé de lutte contre les champignons phytopathogènes, caractérisé en ce que l'on traite les champignons ou les surfaces, plantes ou semences risquant d'être infestées par des champignons avec une quantité efficace du point de vue fongicide d'un composé de la formule I selon la revendication 1.

4. Procédé de préparation de dérivés de la pyridazinone de la formule I selon la revendication 1, caractérisé en ce que l'on fait réagir une 4,5-dihalo-pyridazine-3(2H)-one de la formule II

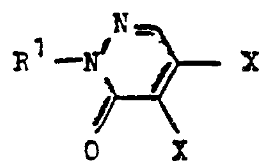

(II)

en présence d'une base avec un mercaptan de la formule III

$$R^2—(CH_2)_n—SH \quad (III),$$

dans lesquelles $R^1$, $R^2$, X et n possèdent les significations définies dans la revendication 1, puis l'on oxyde le dérivé de 5-thio-pyridazine-3(2H)-one obtenu de la formule IV

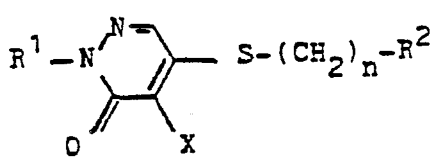

(IV)

en un composé de la formule I.

5. Procédé suivant la revendication 4, caractérisé en ce que l'on fait réagir un composé de la formule II

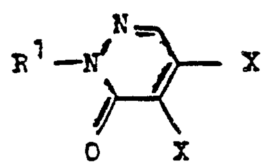

(II)

et un mercaptan de la formule III

$$R^2—(CH_2)_n—SH \quad (III),$$

dans lesquelles $R^1$, $R^2$, X et n possèdent la signification définie dans la revendication 1, à des températures comprises entre — 40 et + 100 °C dans un solvant non nucléophile et en présence d'une amine.